# DEMANDE DE BREVET EUROPEEN

(11) **EP 3 421 095 A1**
(43) Date de publication de la demande: **02.01.2019**
(21) Numéro de dépôt: 18181247.0
(22) Date de dépôt: 02.07.2018
(51) Int. Cl.: A61Q 3/02, A61K 8/73, A61K 8/97

(54) **VERNIS A ONGLES COMPRENANT UN OPACIFIANT D'ORIGINE VEGETALE**

(30) Priorité: 30.06.2017 FR 1756173
(71) Demandeur: Hyteck, 75006 Paris (FR)
(72) Inventeur: RUBERTI, Pascale, 13122 VENTABREN (FR); VAUSSELIN, Anne, 84560 MENERBES (FR)
(74) Mandataire: Tripoz, Inès

(57) **Abrégé**

L'invention concerne une formulation de vernis à ongles, comprenant au moins un opacifiant caractérisé en ce que ledit au moins un opacifiant est d'origine végétale.

Dans un mode de réalisation, l'invention concerne une formulation exempte d'opacifiant d'origine minérale comme le dioxyde de titane, le dioxyde de zinc ou le talc.

Dans un mode de réalisation, l'invention concerne une formulation qui comprend un pigment ou colorant choisi dans le groupe des pigments ou colorants d'origine végétale et une base exempte de filtre UV.

## Description

La présente invention est relative au domaine cosmétique, et plus particulièrement au domaine des vernis à ongles.

La formulation d'une composition de vernis à ongles est relativement complexe, car elle nécessite un assemblage précis de différents ingrédients.

Le changement d'un ou plusieurs ingrédients peut véritablement affecter les propriétés du vernis, ce qui explique que les compositions de vernis à ongles disponibles actuellement sur le marché présentent une grande similitude.

Généralement, deux grands types d'ingrédients sont utilisés dans les compositions de vernis à ongles : les ingrédients destinés à rester sur l'ongle après séchage, et les ingrédients destinés à s'évaporer après l'application du vernis.

Les vernis à ongles doivent présenter des caractéristiques techniques bien précises.

En particulier, il s'agit de la tenue du vernis, de la brillance du vernis, de la facilité d'application du vernis, ainsi que du temps de séchage.

Par ailleurs, la stabilité ainsi que l'innocuité des ingrédients est aussi très importante. La recherche d'ingrédients non toxiques et leur substitution aux ingrédients classiques est une recherche essentielle car la stabilité, les propriétés rhéologiques et de tenue doivent être préservées.

Parmi les composés, très controversés quant à leur innocuité, contenus dans les vernis à ongles, on peut citer les solvants, les filtres UV, les pigments ou colorants et opacifiants.

S'agissant des solvants la plupart aujourd'hui peuvent être biosourcés et un choix judicieux de ceux-ci permet de préserver l'environnement.

Les résines et les filmogènes peuvent également être biosourcés.

Les problèmes à résoudre sont actuellement la possibilité de biosourcer les pigments et les colorants pour éviter l'utilisation de colorants ou de pigments ou colorants toxiques.

Il existe également un problème pour la stabilisation et la protection desdits colorants ou pigments de la dégradation par les UV qui conduit à une modification des teintes, à une décoloration ou même un changement de teinte au fil du temps de stockage et/ou après application.

Actuellement sont utilisés des filtres UV organiques or, les filtres UV organiques sont pour la plupart des perturbateurs endocriniens leur utilisation doit donc être limitée.

La plupart des vernis exempts de filtres UV comprennent des pigments ou colorants chimiques qui sont stables. Il n'existe pas aujourd'hui de formulations de vernis à ongles exempts de filtres UV chimique et/ou minéral comprenant des pigments ou colorants naturels d'origine végétale.

De façon surprenante, la demanderesse a résolu le problème de l'utilisation des opacifiants d'origine minérale comme le dioxyde de titane en lui substituant des matières organiques pulvérulentes non solubles dans les bases et résines utilisées, lesdites matières étant des poudres d'origine végétale, des poudres de polysaccharides et plus particulièrement de poudre de mannane.

De façon également surprenante la demanderesse a résolu le problème de la stabilisation des couleurs des pigments et colorants en utilisant des mélanges de pigments ou colorants issus de plantes notamment sur base huileuse ou aqueuse et/ou en utilisant des pigments ou colorants encapsulés comme ceux décrits dans la demande de brevet WO2013/156863 au nom de E.P.C..

La présente invention concerne ainsi une formulation de vernis à ongles, comprenant au moins un opacifiant caractérisé en ce que ledit au moins un opacifiant est d'origine végétale.

Dans un mode de réalisation le vernis selon l'invention est exempt d'opacifiant d'origine minérale comme le dioxyde de titane, le dioxyde de zinc ou le talc.

Dans un mode de réalisation, ledit au moins un opacifiant d'origine végétale est un polysaccharide sous forme pulvérulente dont la granulométrie est inférieure à 10 µm.

Formulation selon l'une quelconque des revendications 1 ou 2, caractérisée en ce que ledit au moins un opacifiant constitué d'un matériau organique sous forme pulvérulenteest un polysaccharide sous forme pulvérulente dont la granulométrie est inférieure à 10 µm.

Formulation selon l'une quelconque des revendications précédente, caractérisée en ce que ledit polysaccharide sous forme pulvérulente est du mannane.

La présente invention concerne également une formulation de vernis à ongles, comprenant au moins un pigment ou colorant d'origine végétale et une base exempte de filtre UV.

Elle concerne plus particulièrement une formulation de vernis à ongles, comprenant une base comprenant au moins entre 50 et 75 % de solvant, entre 10 et 20 % de filmogène, entre 5 et 15 % de résine et entre 2 et 6 % de plastifiant caratérisée en ce qu'elle comprend au moins un opacifiant constitué d'un matériau organique sous forme pulvérulente.

Dans un mode de réalisation, l'invention concerne une formulation de vernis à ongles, comprenant au moins un opacifiant caractérisé en ce que ledit au moins un opacifiant est d'origine végétale.

Dans un mode de réalisation, ladite formulation est exempte d'opacifiant d'origine minérale comme le dioxyde de titane, le dioxyde de zinc ou le talc.

Formulation selon la revendication 1, caractérisée en ce qu'elle est exempte d'opacifiant constitué d'un matériau minéral sous forme pulvérulente.

Dans un mode de réalisation, ladite formulation est caractérisée en ce que ledit au moins un opacifiant d'origine végétale est un polysaccharide sous forme pulvérulente dont la granulométrie est inférieure à 10 µm.

Dans un mode de réalisation, ladite formulation est caractérisée en ce que ledit au moins un opacifiant d'origine végétale est un polysaccharide sous forme pulvérulente dont la granulométrie est inférieure à 8 µm.

Dans un mode de réalisation, ladite formulation est caractérisée en ce que ledit au moins un opacifiant d'origine végétale est un polysaccharide sous forme pulvérulente dont la granulométrie est inférieure à 5 µm.

Dans un mode de réalisation, ladite formulation est caractérisée en ce que ledit au moins un opacifiant d'origine végétale est un polysaccharide sous forme pulvérulente dont la granulométrie est comprise entre 2 et 5 µm.

Dans un mode de réalisation, ladite formulation est caractérisée en ce que ledit au moins un opacifiant d'origine végétale est un polysaccharide sous forme pulvérulente dont la granulométrie est de 3 µm.

Dans un mode de réalisation, ladite formulation est caractérisée en ce qu'elle est exempte de filtre UV chimique.

Dans un mode de réalisation, ladite formulation est caractérisée en ce qu'elle comprend au moins un pigment ou colorant choisi dans le groupe des pigments ou colorants organique d'origine.

Dans un mode de réalisation, ladite formulation est caractérisée en ce que ledit au moins un pigment ou colorant organique d'origine végétale est un pigment ou colorant encapsulé.

Dans un mode de réalisation, ladite formulation est caractérisée en ce que ledit au moins un pigment ou colorant organique d'origine végétale est un pigment ou colorant en solution dans une huile.

Dans un mode de réalisation, ladite formulation est caractérisée en ce que ledit au moins un pigment ou colorant organique d'origine végétale est un pigment ou colorant en solution dans l'eau.

Dans un mode de réalisation, ladite formulation est caractérisée en ce qu'elle comporte en outre au moins un actif.

Dans un mode de réalisation, ladite formulation est caractérisée en ce que la proportion pondérale du au moins un opacifiant est comprise entre 2 et 15 % en poids total de la formulation.

Dans un mode de réalisation, ladite formulation est caractérisée en ce que la proportion pondérale du au moins un pigment ou colorant organique d'origine végétale est comprise entre 0,1 et 8 % en poids total de la formulation.

Dans un mode de réalisation, ladite formulation est caractérisée en ce que ledit au moins un polysaccharide sous forme pulvérulente est du mannane.

Dans un mode de réalisation, ladite formulation est caractérisée en ce que la proportion pondérale du au moins un opacifiant est comprise entre 2 et 15 % en poids total de la formulation.

Dans un mode de réalisation, ladite formulation est caractérisée en ce que la proportion pondérale du au moins un opacifiant est comprise entre 2 et 11 % en poids total de la formulation

Dans un mode de réalisation, ladite formulation est caractérisée en ce que la proportion pondérale du au moins un opacifiant est comprise entre 3 et 8 % en poids total de la formulation

Dans un mode de réalisation, ladite formulation est caractérisée en ce qu'elle comprend au moins un pigment ou colorant choisi dans le groupe des pigments ou colorants d'origine végétale et une base exempte de filtre UV.

Dans un mode de réalisation, ladite formulation est caractérisée en ce que ledit au moins un pigment ou colorant d'origine végétale est un pigment ou colorant encapsulé.

Dans un mode de réalisation, ladite formulation est caractérisée en ce que le pigment ou colorant encapsulé est choisi parmi les pigments ou colorants décrits dans la demande de brevet WO2013/156863 au nom de E.P.C., à savoir un colorant encapsulé dans un polysaccharide, notamment un amidon ou ses dérivés, un glycogène, une cellulose ou ses dérivés, une chitine ou une pectine.

Dans un mode de réalisation, ladite formulation est caractérisée en ce que ledit au moins un pigment ou colorant d'origine végétale est un pigment ou colorant en solution dans une huile.

Dans un mode de réalisation, ladite formulation est caractérisée en ce que ledit au moins un pigment ou colorant d'origine végétale est un pigment ou colorant en solution dans l'eau.

Dans un mode de réalisation, ladite formulation est caractérisée en ce qu'elle comporte en outre au moins un actif.

Dans un mode de réalisation, ladite formulation est caractérisée en ce que la proportion pondérale du au moins un pigment ou colorant est comprise entre 0,1 et 15 % en poids total de la formulation.

Dans un mode de réalisation, ladite formulation est caractérisée en ce que la proportion pondérale du au moins un pigment ou colorant est comprise entre 0,3 et 10 % en poids total de la formulation.

Dans un mode de réalisation, ladite formulation est caractérisée en ce que la proportion pondérale du au moins un pigment ou colorant est comprise entre 0,5 et 5 % en poids total de la formulation.

Les définitions suivantes permettent de mieux comprendre l'invention.

### Vernis à ongles :

Un produit cosmétique destiné à embellir, à protéger, à soigner l'ongle ou à en masquer certains défauts.

### Solvant :

Toute molécule volatile qui permet lors de son évaporation le séchage du vernis. Les solvants les plus utilisés sont l'acétate de butyle et l'acétate d'éthyle. Généralement la matrice « solvants» représente 70% du poids total d'une formule. Le temps de séchage d'un vernis dépend de la vitesse d'évaporation du solvant et de la nature de la matrice dans laquelle il est inséré.

### Agro-solvants :

On nomme « agro-solvants », les solvants naturels obtenus à partir de céréales ou tubercules par fermentation des sucres en alcools et acides, qui, par des procédés de chimie verte, sont transformés en solvants d'origine 100% naturelle. **Agent filmogène** :

Son rôle est de constituer une pellicule souple et brillante qui adhère à la surface de l'ongle.

Les résines sont des agents filmogènes. Les résines utilisées sont par exemple des résines arylsulfonamide, des copolymères acryliques ou des polyesters. Compte tenu de leur forte viscosité et afin de faciliter leur mise en oeuvre, les résines sont généralement mises en solution dans un solvant, généralement l'acétate de butyle pour application en vernis cosmétique. Dans tous les cas, elles doivent être compatibles avec les autres constituants dont la nitrocellulose.

La nitrocellulose, agent filmogène principal des vernis, est utilisée sous forme d'un solide pulvérulent imbibé d'un mouillant aqueux ou alcoolique pour des raisons de sécurité. La nitrocellulose résulte de la nitration de la cellulose, obtenue principalement à partir de linters de coton, dans le cadre d'une application cosmétique. L'origine renouvelable de la nitrocellulose en fait une matière première intéressante pour la valorisation de la biomasse et la formulation de vernis à ongles respectueux de l'environ nement.

### Plastifiant :

Les plastifiants sont des composés non volatils qui apportent de la souplesse au film pour lui permettre de s'adapter aux flexions de l'ongle. Les plastifiants agissent principalement sur la dureté du film et sur la tenue du vernis. En effet, un film trop mou s'userait trop rapidement alors qu'un film trop dur pourrait s'écailler au moindre choc. L'ajustement des teneurs en nitrocellulose, résines et plastifiants permet d'obtenir un film aux propriétés adaptées (dureté, tenue,...).

### Agent thixotropant / rhéologique :

Les agents thixotropants, bien que présents en faible quantité dans la composition finale, ont un rôle essentiel. Ils apportent des propriétés rhéologiques qui permettent de maintenir en suspension les nacres et pigments afin de minimiser les risques de sédimentation.

Les composés utilisés sont généralement des argiles bentonites ou hectorites.

### Charge :

Dans une formulation, une charge est une substance solide, non miscible et dispersée par un moyen mécanique dans une matrice. Elle est introduite dans un mélange pour améliorer certaines propriétés, par exemple, le pouvoir couvrant, de réflexion ou d'adhésion d'une peinture ou modifier la densité et éventuellement diminuer le coût.

### Pigments ou colorants :

Les pigments sont des matières pulvérulentes colorées qui ne se dissolvent pas dans le milieu dans lequel ils sont introduit. Ils doivent être répartis soigneusement dans le milieu. Les caractéristiques de la composition, telles que la couleur, le pouvoir colorant, l'opacité / la transparence et résistance à la lumière, sont déterminées par le type de pigment.

Quand une substance colorante se dissout dans le milieu dans lequel elle est introduite elle est traditionnellement qualifiée de "colorant" et non pas "pigment".

Les caractéristiques d'opacité et de transparence des pigments sont uniquement percevables lorsque le milieu dans lequel ils sont introduits ne contient pas de charges opaques.

Le degré d'opacité ou bien de transparence varie selon le type de pigment.

Les pigments sont ainsi constitués des particules solides de très petite taille (quelques micromètres de diamètre) qui ne se solubilisent pas et doivent rester en suspension dans le vernis. Ils peuvent être classés en trois familles :
- les pigments minéraux de type oxydes métalliques (par exemple, Fe₂O₃ de couleur rouge ou TiO₂ de couleur blanche qui est également utilisé comme opacifiant ou charge opaque). Ces pigments possèdent un excellent pouvoir couvrant mais une densité élevée.
- les pigments ou colorants organiques synthétiques, qui possèdent un pouvoir couvrant plus limité et qui sont lorsqu'ils sont solubles dans les bases requièrent l'utilisation d'un opacifiant pour obtenir un effet couvrant. L'éventail de couleurs proposé par les fabricants de pigments est très large.
- les pigments ou colorants organiques d'origine végétale. Il s'agit de molécules extraites de végétaux, qui sont soient greffées sur un support poudreux d'origine minérale ou végétale soient mises en solution dans un support liquide (huileux, aqueux, solvanté, glycériné). La palette de couleurs existantes est restreinte et leur résistance à l'oxydation est limitée sous l'effet des rayonnements ultraviolets et de la température. Les grandes familles de pigments d'origine végétale comprennent les chlorophylles, les caroténoïdes, les flavonoîdes, les bétalaïnes et les anthocyanes. Là encore en fonction de leur solubilité dans le milieu et ou leur pouvoir couvrant, il va être nécessaire d'utiliser une charge opaque ou opacifiant.

### Polysaccharide :

Polymères constitués de plusieurs oses liés entre eux par des liaisons osidiques. Formule générale : [Cx(H2O)y)]n-(où y est généralement x - 1).

### Mannane :

Polysaccharide composé principalement de monomères de mannose liés entre eux par des liaisons osidiques. Il est aussi appelé D-mannosan et son numéro CAS est le 9036-88-8.

### Encapsulation :

En chimie et en pharmacie galénique, l'encapsulation consiste à enfermer une substance dans une capsule.

L'objectif est :
- soit de protéger la substance contre des interactions avec le milieu extérieur. On encapsule par exemple certains filtres UV contenus dans les crèmes solaires pour les stabiliser et les empêcher d'irriter la peau.
- soit de transporter la substance jusqu'à l'endroit où elle sera libérée pour agir. Les liposomes par exemple sont des sphères utilisées pour transporter les substances actives des cosmétiques dans la peau.

Selon la taille des capsules, il peut s'agir de microencapsulation (dimension de l'ordre du micromètre) ou de nano-encapsulation (dimension de l'ordre du nanomètre). Les capsules sont alors appelées « molécules-cages ».

### Filtre UV :

Un composé chimique qui filtre les ondes électromagnétiques et bloque ou absorbe les radiations ultraviolettes. Il existe deux types de filtres ultraviolets : les filtres chimiques et les filtres minéraux.

Les filtres chimiques autorisés sont au nombre de 25, ils sont choisis dans la liste suivante :
Camphor benzalkonium Methosulfate, Homosalate, 3-Benzophénone ou (Oxybenzone), Phenylbenzimidazole - Sulfonic Acid ou (Ensulizole), erephthalylidene Dicamphor Sulfonic Acid ou (Ecamsul ou Meroxyl SX), Butyl Methoxydibenzoylmethane ou (Avobenzone), Benzylidene Camphor Sulfonic Acid, Octocrylène, Polyacrylamidomethyl Benzylidène Camphor, Ethylhexyl Methoxycinnamate (Octinoxate), PEG 25-PABA, Isomamyl p- Methoxycinnamate, Ethylhexyl Triazone (Octyl Triazone), Drometrizole Trisiloxane (Meroxyl XL), Diethylhexyl Butamido Triazone, 4-Methylbenzylidene Camphor, 3-Benzylidène Camphor, Ethylhexyl Salicylate (INCI) ou Octyl Salicylate (Octisalate), Ethylhexyl Dimethyl PABA ou Octyl Dimethyl PABA (Padimate O), Benzophénone-4, Benzophénone-5 ou Sulisobenzone (Benzophenone-4), Methylène Bis- Benzotriazolyl Tetra- methylbutylphenol (Trinosorb M), Disodium Phenyl Dibenzimidazole Tetrasulfonate, Bis-Ethylhexyloxyphenol Methoxyphenyl Triazine (Trinosorb S), Polysilicone-15 et Diethylamino Hydroxybenzoyl Hexyl Benzoate.

Le seul filtre UV minéral autorisé en cosmétique est le dioxyde de titane.

Dans un mode de réalisation les formulations comprennent une base comprenant au moins entre 50 et 75 % de solvant, entre 10 et 20 % de filmogène, entre 5 et 15 % de résine, entre 2 et 6 % de plastifiant.

Elles peuvent en outre comprendre, entre 2 et 10% d'agent rhéologique et moins de 5 % de filtres UV, opacifiants, tampons, actifs divers.

Dans la base sont ensuite dispersés des pigments ou colorants dans des proportions pondérales qui varient entre 0,1 et 15 % et un ou des opacifiants entre 0,1 et 15 %.

Dans la base sont ensuite dispersés des pigments ou colorants dans des proportions pondérales qui varient entre 0,3 et 10 % et un ou des opacifiants entre 1 et 10%.

Dans la base sont ensuite dispersés des pigments ou colorants dans des proportions pondérales qui varient entre 0,5 et 5 % et un ou des opacifiants entre 2 et 8%.

L'invention est illustrée par les exemples suivants.

### I. Formulations de bases de vernis à ongles

**Contre-exemple Base 1 avec filtre UV**

| Fonction | Composants | Composition massique (%) |
|---|---|---|
| Solvant | Acétate de butyle | 42 |
| Solvant | Acétate d'éthyle | 24,6 |
| Filmogène | Nitrocellulose | 17,5 |
| Résine | Résine Tosylamide/epoxy | 6,4 |
| Agent rhéoloqique | Alcool Isopropyle | 6,3 |
| Agent rhéologique | Stearalkonium hectorite | 2 |
| Filtre UV | Benzophenone-1 | 1 |
| Opacifiant | Copolymère de Styrène/acrylate | 0,02 |
| Tampon | Acide malique | 0,06 |
| Tampon | Acide citrique | 0,07 |
| Filtre UV | Ethyl trimethylbenzoyl phenylphosphinate | 0,05 |

**Base 2 sans filtre UV**

| Fonction | Composants | Composition massique (%) |
|---|---|---|
| Solvant | Acétate de butyle | 41,85 |
| Solvant | Acétate d'éthyle | 24,82 |
| Filmogène | Nitrocellulose | 17,8 |
| Résine | Résine Tosylamide/epoxy | 6,57 |
| Agent rhéologique | Alcool Isopropyle | 6,47 |
| Agent rhéoloqique | Stearalkonium hectorite | 2,22 |
| Opacifiant | Copolymère de Styrene/acrylate | 0,12 |
| Tampon | Acide malique | 0,07 |
| Tampon | Acide citrique | 0,08 |

**Base 3 sans filtre UV**

| Fonction | Composants | Composition massique (%) |
|---|---|---|
| Solvant | Acétate d'éthyle | 51 |
| Solvant | Acétate de butyle | 21,2 |
| Filmoqène | Nitrocellulose | 12,8 |
| Plastifiant | Citrate d'Acétyle tributyle | 6 |
| Résine | Résine polyester | 9 |

### II. Formulations de vernis à ongles sans filtre UV avec pigments ou colorants végétaux non encapsulés majoritaires

A partir des bases de vernis à ongles décrites en I, des formulations de vernis à ongles sont préparées.

Les formulations de vernis à ongles sont préparées selon le protocole suivant. L'opacifiant et les pigments ou colorants sont introduits dans une coupelle, 10% de la base est ajouté dans la coupelle, puis après mélange le reste de base est ajouté et mélangé, quand le mélange est homogène, le vernis obtenu est transvasé dans le flacon à vernis à ongles sans attendre.

**Vernis à ongles II1**

| Fonction | Composants | Composition massique (%) |
|---|---|---|
| Base | Base 2 | 85 |
| Opacifiant | Mannane | 6 |
| Piqment encapsulé | Pourpre sureau | 2 |
| Pigment encapsulé | Patate douce | 2 |
| Pigment non encapsulé | Campo siddha neer kaddal neelam | 5 |

**Vernis à ongles II2**

| Fonction | Composants | Composition massique (%) |
|---|---|---|
| Base | Base 3 | 87,5 |
| Opacifiant | Mannane | 8 |
| Pigment encapsulé | Rose patate | 1 |
| Pigment non encapsulé | Extrait CO2 de Lithospermum | 0,5 |
| Pigment non encapsulé | Campo siddha neer ellam manjal | 3 |

### III. Formulations de vernis à ongles sans filtre UV avec pigments ou colorants végétaux encapsulés majoritaires

Les formulations de vernis à ongles sont préparées selon le protocole suivant. L'opacifiant et les pigments ou colorants sont introduits dans une coupelle, 10% de la base est ajouté dans la coupelle, puis après mélange le reste de base est ajouté et mélangé, quand le mélange est homogène, le vernis obtenu est transvasé dans le flacon à vernis à ongles sans attendre.

**Vernis à ongles III1**

| Fonction | Composants | Composition massique (%) |
|---|---|---|
| Base | Base 2 | 88,25 |
| Opacifiant | Mannane | 3,00 |
| Pigment non encapsulé | Extrait CO2 de Lithospermum | 0,50 |
| Pigment encapsulé | Rouge radis | 8,00 |
| Actif | Gomme de pistacia lentiscus et tryglycéride capric/aprylique | 0,25 |

**Vernis à ongles III2**

| Fonction | Composants | Composition massique (%) |
|---|---|---|
| Base | Base 3 | 92,15 |
| Opacifiant | Mannane | 3,00 |
| Pigment encapsulé | Rose patate douce | 4,00 |
| Pigment non encapsulé | Extrait CO2 de Lithospermum | 0,60 |
| Actif | Gomme de pistacia lentiscus et tryqlvcéride capric/aprylique | 0,25 |

**Vernis à ongles III3**

| Fonction | Composants | Composition massique (%) |
|---|---|---|
| Base | Base 3 | 86,75 |
| Opacifiant | Mannane | 4,00 |
| Pigment encapsulé | Pourpre sureau | 6,00 |
| Pigment encapsulé | Rose patate | 3,00 |
| Actif | Gomme de pistacia lentiscus et tryglycéride capric/aprylique | 0,25 |

### IV. Pouvoir opacifiant du mannane et stabilité de la dispersion

### A) Comparaison avec le TiO₂

Le pouvoir opacifiant du mannane est vérifié en comparant après application sur les ongles des formulations ci-dessus décrites comprenant du mannane.

On obtient avec les formulations comprenant du mannane une opacification équivalente à celle obtenue avec des formulations du commerce comprenant des pigments ou colorants chimiques et du TiO₂.

La stabilité de la dispersion est également vérifiée, à savoir la décantation et la possibilité d'homogénéiser la dispersion avec des dispersions de mannane à 10 % dans les bases ci-dessus décrites.

Les bases comprenant 10% de mannane sont portées à 45°C et à 4°C. Elles sont laissées reposer. Puis la décantation et la faculté de remise en suspension sont vérifiées.

Quatre propriétés sont testées, la couvrance, c'est-à-dire le pouvoir opacifiant ou couvrant, le temps de séchage, la tenue et la brillance.

Les résultats obtenus sont équivalents à ceux obtenus avec des suspensions de TiO₂ de granulométries comparables, à savoir inférieures ou égales à 10 µm. Les essais sont répétés en présence de pigments ou colorants et les résultats obtenus sont également équivalents avec ceux observés avec des formulations du commerce comprenant des pigments ou colorants chimiques et du TiO₂.

### B) Tests comparatifs

Des tests comparatifs ont été effectués en comparaison avec d'autres charges organiques de granulométries comparables.

Les résultats obtenus sont rassemblés dans le tableau suivant :

| | | Résultat des essais * | | | | |
|---|---|---|---|---|---|---|
| Composé | INCI | Concentration dans formule (% massique) | Couvrance | Temps de séchage | Tenue | Brillance |
| Reisita natural | oryza sativa starch | 10% | ++ | ++ | + | +++ |
| | | 20% | | | | |
| Amidon de maïs BIO | Zea mays starch | 10% | + | ++ | ++ | + |
| | | 20% | | | | |
| QUEMINA 21.257 | zea mays (corn) starch | 10% | + | ++ | ++ | + |
| | | 20% | | | | |
| Arrow root powder | Fécule très riche en amidon | 10% | ++ | ++ | ++ | + |
| | | 20% | | | | |
| Tapioca natural | Tapioca starch | 10% | ++ | +++ | ++ | + |
| | | 20% | | | | |
| EP Cannan (Mannan) | Mannan | 3% | +++ | ++++ | ++++ | +++ |
| | | 5% | | | | |
| | | 10% | | | | |

Seul le mannane permet d'obtenir des résultats satisfaisants tant en couvrance, temps de séchage, qu'en tenue et brillance et ce en en introduisant moins de 10% dans la formulation.

### V. Tests de stabilité aux UV de pigments ou colorants végétaux dans une base de vernis à ongles sans filtre UV

Pour tester la tenue des pigments ou colorants végétaux susceptibles d'être utilisés dans une formule de vernis à ongles, il est nécessaire de réaliser des tests de stabilité aux UV afin de valider la stabilité de la couleur dans le temps.

### Conditions expérimentales

Les tests de stabilité sont initiés le jour de la fabrication de la formulation (J0). Ces tests sont effectués à la lumière naturelle.

### Réalisation des échantillons à tester

6 ml de la formulation de vernis à ongles à tester sont fabriqués puis transférés dans un flacon vernis de 6 ml.

### Test de stabilité

Une goutte de la formulation à tester est déposée sur une feuille de papier blanche, à J0. La feuille est ensuite réservée dans un espace à l'abri de la lumière. En parallèle, le flacon est placé à la lumière naturelle, à partir de J0.

### Contrôle de stabilité

Une goutte de la formulation à tester est déposée sur la feuille de papier blanche utilisé à J0.

Ce contrôle de stabilité est effectué aux fréquences suivantes : J+7, J+14, M+1.

### Exploitation des résultats

Les gouttes déposées sur la feuille de papier blanche sont comparées les unes aux autres afin de déterminer l'évolution de la couleur au cours du temps.

Les formulations suivantes sont testées :

**Formulation V1**

| Fonction | Composants | Composition massique (%) |
|---|---|---|
| Base | Base 2 | 90 |
| Pigment non encapsulé | Eclipta Prostrata (extrait) | 8,1 |
| Pigment non encapsulé | Melia Azadirachta (extrait de feuilles) | 1,6 |
| Pigment non encapsulé | Moringa Oleifera (huile de graines) | 0,3 |

**Formulation V2**

| Fonction | Composants | Composition massique (%) |
|---|---|---|
| Base | Base 2 | 90 |
| Pigment non encapsulé | Melia Azadirachta (extrait de feuilles) | 9,5 |
| Pigment non encapsulé | Moringa Oleifera (huile de graines) | 0,5 |

**Formulation V3**

| Fonction | Composants | Composition massique (%) |
|---|---|---|
| Base | Base 3 | 10 |
| Pigment | Melia Azadirachta (extrait) | 5 |
| Pigment non encapsulé | Melia Azadirachta (extrait de feuilles) | 4,5 |
| Pigment non encapsulé | Moringa Oleifera (huile de qraines) | 0,5 |

**Formulation V4**

| Fonction | Composants | Composition massique (%) |
|---|---|---|
| Base | Base 3 | 90 |
| Pigment non encapsulé | Melia Azadirachta (extrait de feuilles) | 3 |
| Pigment non encapsulé | Melia Azadirachta (extrait de fleurs) | 2,5 |
| Pigment non encapsulé | Corallina officinalis (extrait) | 2,2 |
| Pigment non encapsulé | Coccinia Indica (extrait de fruits) | 0,5 |
| Pigment non encapsulé | Poudre d'ambre | 0,5 |
| Pigment non encapsulé | Solanum Melonqena (extrait de fruits) | 0,5 |
| Pigment non encapsulé | Ocimum Sanctum (extrait de feuilles) | 0,3 |
| Pigment non encapsulé | Curcuma Longa (extrait de feuilles) | 0,3 |
| Pigment non encapsulé | Moringa Oleifera (huile de qraines) | 0,2 |

**Formulation V5**

| Fonction | Composants | Composition massique (%) |
|---|---|---|
| Base | Base 2 | 90 |
| Pigment non encapsulé | Melia Azadirachta (extrait de feuilles) | 3 |
| Pigment non encapsulé | Melia Azadirachta (extrait de fleurs) | 2,5 |
| Pigment non encapsulé | Corallina officinalis (extrait) | 2,25 |
| Pigment non encapsulé | Eclipta Prostrata (extrait) | 1,5 |
| Pigment non encapsulé | Ocimum Sanctum (extrait de feuilles) | 0,5 |
| Pigment non encapsulé | Moringa Oleifera (huile de graines) | 0,25 |

**Formulation V6**

| Fonction | Composants | Composition massique (%) |
|---|---|---|
| Base | Base 3 | 90 |
| Pigment non encapsulé | Melia Azadirachta (extrait de feuilles) | 3 |
| Pigment non encapsulé | Melia Azadirachta (extrait de fleurs) | 2,5 |
| Pigment non encapsulé | Corallina officinalis (extrait) | 2,1 |
| Pigment non encapsulé | Coccinia Indica (extrait de fruits) | 1 |
| Pigment non encapsulé | Solanum Melongena (extrait de fruits) | 0,5 |
| Pigment non encapsulé | Aloe Bardensis (extrait de fleurs) | 0,5 |
| Pigment non encapsulé | Ocimum Sanctum (extrait de feuilles) | 0,1 |
| Pigment non encapsulé | Ocimum Basilicum (extrait de fleurs et de feuilles) | 0,1 |
| Pigment non encapsulé | Curcuma Longa (extrait de feuilles) | 0,1 |
| Diluant | Eau | 0,1 |

**Formulation V7**

| Fonction | Composants | Composition massique (%) |
|---|---|---|
| Base | Base 3 | 90,0 |
| Pigment non encapsulé | Melia Azadirachta (extrait de fleurs) | 3,2 |
| Pigment non encapsulé | Melia Azadirachta (extrait de feuilles) | 2,0 |
| Pigment non encapsulé | Ocimum Sanctum (extrait de feuilles) | 1,8 |
| Pigment non encapsulé | Ocimum Basilicum (extrait) | 1,0 |
| Pigment non encapsulé | Curcuma Longa (extrait de feuilles) | 1,0 |
| Pigment non encapsulé | Corallina officinalis (extrait) | 0,8 |
| Diluant | Eau | 0,2 |

Les résultats obtenus sont présentés dans le tableau suivant :

| Formulations | J + 7 | J + 14 | M + 1 |
|---|---|---|---|
| V1 | Pas de décoloration | Pas de décoloration | Pas de décoloration |
| V2 | Pas de décoloration | Pas de décoloration | Pas de décoloration |
| V3 | Pas de décoloration | Pas de décoloration | Pas de décoloration |
| V4 | Pas de décoloration | Pas de décoloration | Pas de décoloration |
| V5 | Pas de décoloration | Pas de décoloration | Pas de décoloration |
| V6 | Pas de décoloration | Pas de décoloration | Pas de décoloration |
| V7 | Pas de décoloration | Pas de décoloration | Pas de décoloration |

En conclusion, les formulations testées présentent une bonne tenue aux UV en l'absence de filtre UV et avec des pigments ou colorants d'origine végétale.

### VI. FABRICATION

Les formulations selon l'invention sont fabriquées en lots de 25 à 200 kg.

Les mélanges sont effectués au moyen d'un agitateur anti-déflagrant dont les vitesses de rotation sont variables de 500 à 1200 rpm en fonction des viscosités des mélanges et de la taille du lot.

La base incolore est introduite dans le réacteur puis les colorants et les additifs sous forme solide comme les opacifiants et/ou les pigments.

L'agitation est maintenue pendant une durée inférieure à 0,5 h en fonction de la taille du lot et de la viscosité du produit.

Après homogénéisation la teinte du produit est contrôlée par comparaison à un contrôle puis la viscosité et les ajustements nécessaires sont effectués.

Lorsque le mélange est conforme le produit est filtré et stocké dans des futs hermétiquement fermés avant la phase de conditionnement en flacons.

## Revendications

1. Formulation de vernis à ongles, comprenant une base comprenant au moins entre 50 et 75 % de solvant, entre 10 et 20 % de filmogène, entre 5 et 15 % de résine et entre 2 et 6 % de plastifiant caratérisée en ce qu'elle comprend au moins un opacifiant constitué d'un matériau organique sous forme pulvérulente.

2. Formulation selon la revendication 1, **caractérisée en ce qu'**elle est exempte d'opacifiant constitué d'un matériau minéral sous forme pulvérulente.

3. Formulation selon l'une quelconque des revendications 1 ou 2, **caractérisée en ce que** ledit au moins un opacifiant constitué d'un matériau organique sous forme pulvérulente est un polysaccharide sous forme pulvérulente dont la granulométrie est inférieure à 10 µm.

4. Formulation selon l'une quelconque des revendications précédente, **caractérisée en ce que** ledit polysaccharide sous forme pulvérulente est du mannane.

5. Formulation selon l'une quelconque des revendications précédente, **caractérisée en ce qu'**elle est exempte de filtre UV chimique.

6. Formulation selon l'une quelconque des revendications précédentes, **caractérisée en ce qu'**elle comprend au moins un pigment ou colorant choisi dans le groupe des pigments ou colorants organique d'origine.

7. Formulation selon la revendication 5, **caractérisée en ce que** ledit au moins un pigment ou colorant organique d'origine végétale est un pigment ou colorant encapsulé.

8. Formulation selon la revendication 5, **caractérisée en ce que** ledit au moins un pigment ou colorant organique d'origine végétale est un pigment ou colorant en solution dans une huile.

9. Formulation selon la revendication 5, **caractérisée en ce que** ledit au moins un pigment ou colorant organique d'origine végétale est un pigment ou colorant en solution dans l'eau.

10. Formulation selon l'une quelconque des revendications précédentes, **caractérisée en ce qu'**elle comporte en outre au moins un actif.

11. Formulation selon l'une quelconque des revendications précédentes, **caractérisée en ce que** la proportion pondérale du au moins un opacifiant est comprise entre 2 et 15 % en poids total de la formulation.

12. Formulation selon l'une quelconque des revendications précédentes, **caractérisée en ce que** la proportion pondérale du au moins un pigment ou colorant organique d'origine végétale est comprise entre 0,1 et 8 % en poids total de la formulation.
